(19) Europäisches Patentamt
European Patent Office
Office européen des brevets

(11) Publication number : **0 499 467 A2**

(12) **EUROPEAN PATENT APPLICATION**

(21) Application number : **92301185.2**

(22) Date of filing : **13.02.92**

(51) Int. Cl.$^5$ : **A61K 31/12,** A61K 31/165, A61K 31/19, A61K 31/195, A61K 31/70

(30) Priority : **13.02.91 GB 9103075**

(43) Date of publication of application : **19.08.92 Bulletin 92/34**

(84) Designated Contracting States : **AT BE CH DE DK ES FR GB GR IT LI LU MC NL PT SE**

(71) Applicant : **RADOPATH LIMITED**
**17 Bond Street, St.Helier**
**Jersey (GB)**

(72) Inventor : **Ayuko, Washington Odur, Dr.**
**25 Sundridge Road, Kingstanding,**
**Birmingham B44 9NY (GB)**

(74) Representative : **Green, Alan James et al**
**Hepworth Lawrence Bryer & Bizley, 2nd Floor,**
**Gate House South, West Gate**
**Harlow, Essex CM20 1JN (GB)**

(54) **Use of quinones in the treatment of cancer or AIDS.**

(57) The invention relates to a pharmaceutical composition comprising at least one quinone and to its use in the treatment of disease, especially cancer and virus infections.

The composition of the invention comprises at least one quinone dissolved or dispersed in a liquid diluent or carrier at a concentration of about $10^3$ moles/litre or below, preferably down to $10^{18}$ moles/litre.

In the composition the at least one quinone is preferably :

An anthraquinone glycoside, more preferably carminic acid.

A compound of the formula :

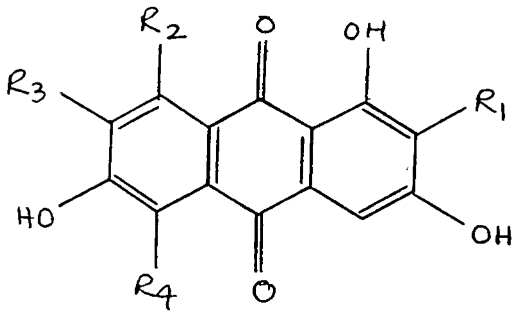

(I)

wherein $R_1$ is hydrogen, a sugar e.g. $-C_6H_{11}O_5$, hydroxy,

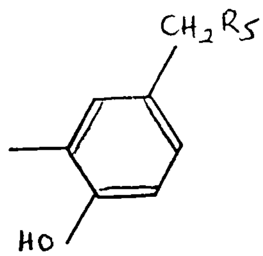

in which $R_5$ is $-CH_2NHAc$, $-CH_2OH$, $-CH(NH_2)CO_2H$ or $-CH_2NH_2$ ; $R_2$ is methyl or $-CO_2H$ ; $R_3$ is hydrogen, $-NH_2$ or $-SO_3X$ in which X is H, Na or K ; and $R_4$ is hydrogen or hydroxy.

Quinhydrone of the formula :

EP 0 499 467 A2

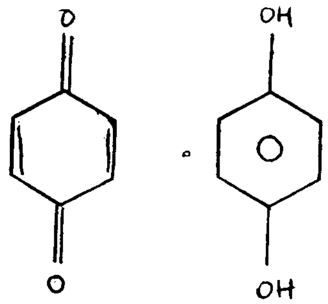

(II)

Tetrahydroxyquinone of the formula :

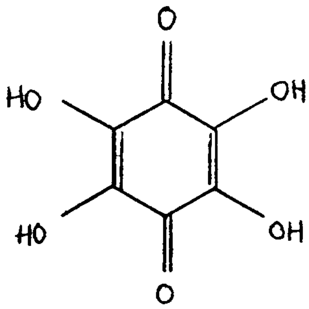

(III)

The present invention relates to a pharmaceutical composition, in particular a composition comprising at least one quinone and to its use in the treatment of disease, especially cancer and virus infections.

Traditional cancer treatments generally involve surgery, radiotherapy, chemotherapy, or some combination thereof. While these treatments are often effective in lengthening a patient's life or sometimes eradicating the cancer, they have well-known serious side effects. More recently, alternative attempts to treat certain cancers have been developed. One such technique, "immunotherapy", is designed to strengthen the innate ability of the patient's immune system to fight cancer.

While diagnostic and treatment techniques have improved significantly over the last decades, there has been very little improvement in the overall survival rate in patients, especially those with solid tumours treated by these orthodox methods. Moreover, although alternative techniques such as "immunotherapy" show promise, the treatments developed thus far can help only a limited number of patients. Also, they still exhibit toxic side effects and/or they are complex and expensive to perform.

In my co-pending International Patent Application No. PCT/GB91/00517 published as WO 91/15200 on 17th October, 1991, and claiming priority, inter alia, from British Patent Application No. 91 03075.9 filed on 13th February, 1991, there is described and claimed for use in the therapy or prophylaxis of neoplasm or viral infection in human and non-human animals, a formulation comprising:

(a) one or more compounds of the general formula:

$$X-P$$

wherein P is trinitrophenyl and X is selected from OH, $NH_2$, halogen, a sulfo group, a carboxyl group, $OCH_3$ or a substituted or unsubstituted hydrazyl group of the formula:

```
Z - N - N -
    |   |
    Y   A
```

wherein A is hydrogen or an unpaired electron of the nitrogen atom, Y is hydrogen or an organic group and Z is an organic group, or Y and Z together with the adjacent nitrogen atom form a nitrogen-containing heterocycle; provided that when X is a substituted or unsubstituted hydrazyl group as aforesaid one of the $NO_2$ groups may be replaced by a sulfo group; and

(b) a quinone, optionally an anthraquinone having a glycosidic moiety, preferably, carminic acid.

Also described and claimed therein are the following, namely:

(A) For use in the therapy of viral infection in human animals, a formulation comprising an anthraquinone glycoside, optionally carminic acid, dissolved in an aqueous medium at a concentration in the range from $10^3$ to $10^{-15}$ moles per litre; and

(B) The use of an anthraquinone glycoside, optionally carminic acid, in the formulation of a medicament for the prophylaxis or treatment of a viral infection.

The present Application also claims priority from British Patent Application No. 91 03075.9 filed on 13th February, 1991, and is directed to that approach to the problem of cancer treatment which involves the use of low concentrations of readily available quinone compounds such as carminic acid (either singularly or in combination). The subject matter described and claimed herein is the original subject matter of Application No. 91 03075.9 which will not be claimed in Patents granted nationally out of the above-mentioned International Application

Thus, the invention the subject of the present Application is based on the surprising and new discovery that quinone compounds are efficacious per se, inter alia, as anticancer and anti-viral agents when administered at low concentrations, essentially regardless of patient bodyweight. Toxicity and expense problems associated with the prior art accordingly do not apply.

Thus, in one aspect the invention provides a pharmaceutical composition, which composition comprises at least one quinone dissolved or dispersed in a liquid diluent or carrier at a concentration of about $10^{-3}$ moles/litre or below.

While the mechanism or mechanisms of the antidisease e.g. antitumour, effect of using a low concentration of about $10^{-3}$ moles/litre or below of at least one quinone in the present invention are not yet entirely clear, it is my belief that they may involve the provision of one or more of the following:

(a) free radicals which can act within the body's cellular structure e.g. a direct cytotoxic action of free radicals and their products;

(b) modulation of the immune system including, for example, the induction of cytokines; and

(c) an effect on the blood supply e.g. tumour blood supply.

In the composition of the invention the or the at least one quinone may be in oxidized or reduced form and

is preferably:

An anthraquinone glycoside, more preferably carminic acid.

A compound of the formula:

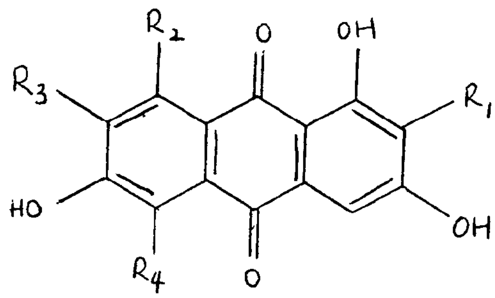

(I)

wherein $R_1$ is hydrogen, a sugar e.g. $-C_6H_{11}O_5$, hydroxy,

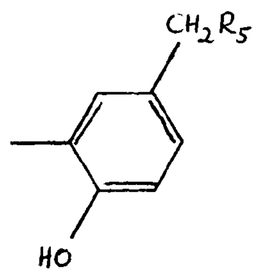

in which $R_5$ is $-CH_2NHAc$, $-CH_2OH$, $-CH(NH_2)CO_2H$ or $-CH_2NH_2$; $R_2$ is methyl or $-CO_2H$; $R_3$ is hydrogen, $-NH_2$ or$SO_3X$ in which X is H, Na or K; and $R_4$ is hydrogen or hydroxy.

Quinhydrone of the formula:

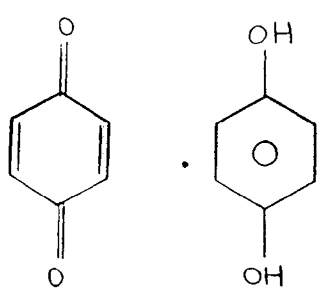

(II)

Tetrahydroxyquinone of the formula:

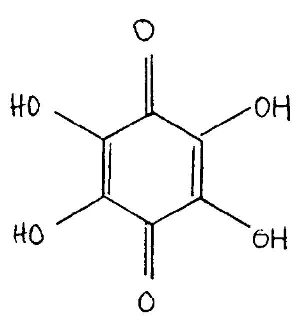

(III)

or a derivative thereof such as:

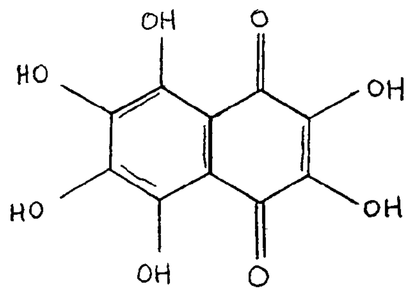

III(a)

2,3,5,6,7,8-hexahydroxy-naphthoquinone
or

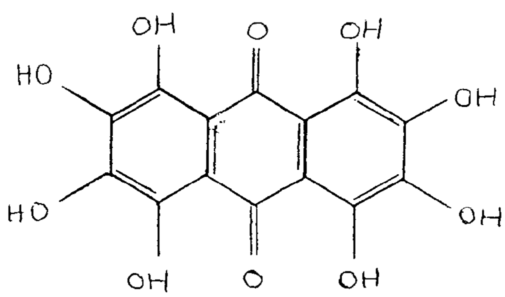

III(b)

1,2,3,4,5,6,7,8 octahydroxyanthraquinone

The preferred compounds within the scope of formula (I) above may be isolated from coccid insects and may be summarised as follows:

Laccaic acid D of the formula:

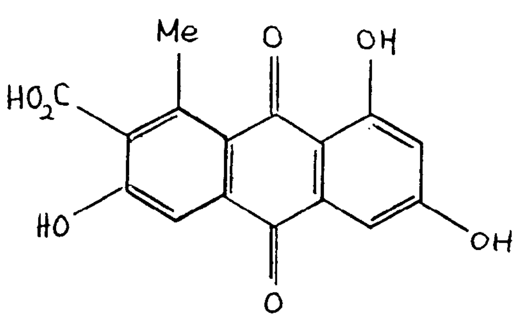

(IV)

Other laccaic acids of the formula:

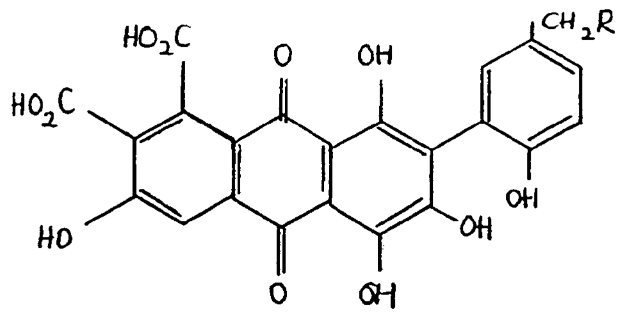

(V)

Laccaic acids

A, R = $CH_2NHAc$

B, R = $CH_2OH$

C, R = $CH(NH_2)CO_2H$

E, R = $CH_2NH_2$

Kermesic acid of the formula:

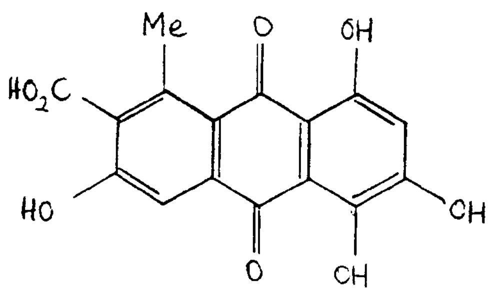

(VI)

Carminic acid of the formula:

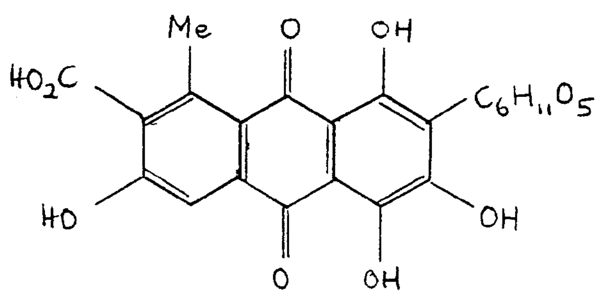

(VII)

Ceroalbolinic acid of the formula:

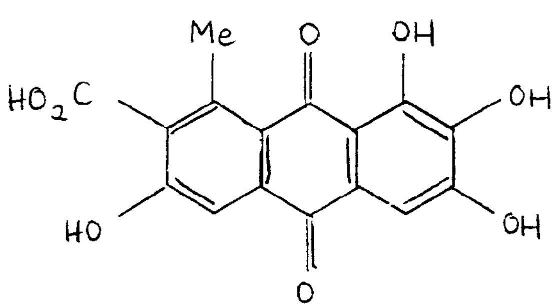

(VIII)

Erythrolaccin of the formula:

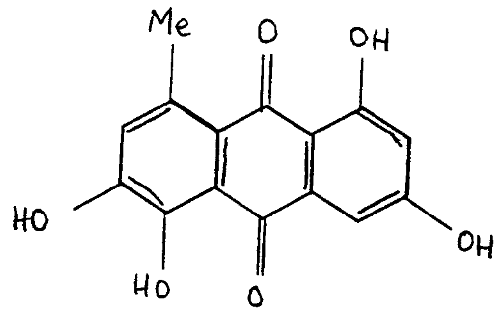

(IX)

Deoxyerythrolaccin of the formula:

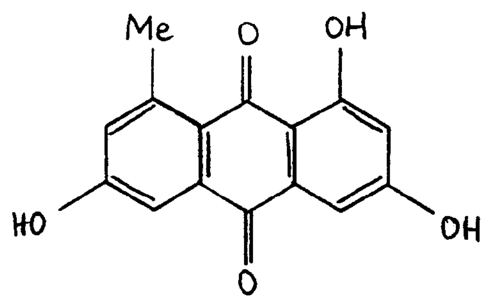

(X)

Thus, generally the invention employs polyhydroxylated quinones which are capable of redox cycling and perhaps also of acting via one or more of the mechanisms mentioned above, for example, initiating and propagating a free radical chain reaction in the host's body at the concentrations as defined.

Preferably the composition of the invention is based on the or the at least one quinone as the sole active ingredient and more preferably on at least two quinones. Preferably also the active compounds e.g. quinones, are present in at least approximately equal amounts.

An especially preferred composition is one comprising carminic acid, quinhydrone and/or tetrahydroquinone. More particular, such a composition comprises approximately equal amounts of carminic acid, quinhydrone and tetrahyroxyquinone.

In the composition of the invention the active quinone compound or compounds each should be present in a low concentration at which free radical reactions take place rather than alkylating reactions. The latter can take place down to $10^{-2}$ moles per litre and thus the compositions of the invention generally employ at least one quinone at a concentration of about $10^{-3}$ moles per litre and lower. At such concentrations the active quinone(s) may initiate free radical endogenous reactions within an animal host. Such reactions selectively kill cells which are cancerous or otherwise diseased such as by virus attack or attack by other microbes or even chemical attack e.g. by antioxidants.

Preferably, the concentration of each quinone is from about
$10^{-3}$ to about $10^{-18}$ moles/litre, more preferably from about
$10^{-3}$ to about $10^{-15}$ moles/litre, most preferably from about
$10^{-6}$ to about $10^{-15}$ moles/litre.

In the composition of the invention the diluent or carrier may be any liquid diluent or carrier which is pharmaceutically acceptable. Thus, the diluent or carrier may be or comprise water, polyethylene glycol, an oil such as arachis oil, or a liquid paraffin.

Preferably, the diluent or carrier is double- or triple-distilled deionized water or polyethylene glycol where slower release is required. While double- or triple-distilled de-ionized water is the preferred solution/suspension liquid, other diluents/carriers such as a dimethylsulfoxide/water solution, arachis oil, olive oil, vegetable oil or corn oil, may be useful as well.

The invention also includes a composition comprising at least one quinone dissolved or dispersed in a pharmaceutically-acceptable liquid medium at a concentration of from about $10^{-3}$ to about $10^{-18}$ moles per litre for use in the therapy or prophylaxis of disease in human and non-human animals

Such a composition is as defined herein and all of said compositions may be administered orally or parenterally e.g. subcutaneously or intramuscularly. Typically, the compounds may be used in a dosage regimen of from about $10^{3}$ moles/kg body weight to about $10^{-18}$ moles per kilogram body weight, although as indicated above their effect is largely independent of body weight. Preferably, a composition for oral use will have a concentration for each quinone of from about $10^{-3}$ to about $10^{-6}$ moles/litre. Alternatively, where use is by a parenteral route each quinone preferably has a lower concentration, typically from about $10^{-9}$ to $10^{-15}$ moles/litre.

The compositions of the invention are particularly useful in the treatment of cancer and virus infections such as AIDS. However, it may also be used to treat bacterial and fungal infections, as well as metabolic and degenerative diseases. In fact, wherever there is cell dysfunction typically caused by antioxidant imbalance within the cell then the compositions of the invention can aid in correcting the said imbalance. Thus, the macro disease manifestations caused by the micro imbalance are brought under control and eventually eliminated by restoring a balance between antioxidant/oxidant functions at the cellular level.

The composition of the invention may be a pharmaceutical or veterinary formulation and may be used in the manufacture of a medicament for the treatment of human or non-human animals. Significantly, carminic acid has been found to exhibit anti-cancer effects when used alone at low molar concentrations according to the invention. Thus, an important aspect of the invention is the use of carminic acid and its derivatives in the preparation of a medicament for the prophylaxis or therapy of cancer or virus infection. Such derivatives have the following general formula:

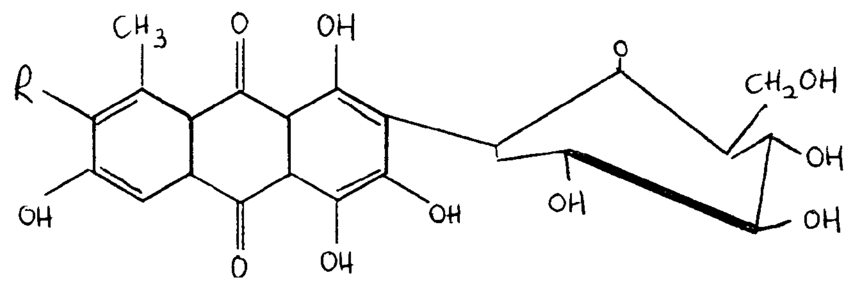

(XI)

where R is COOH (i.e. carminic acid) or some other organic or inorganic functional group such as $NH_2$, $SO_3$ [K, H, Na], and the C-glycoside is any sugar. Also, the anthraquinone may optionally be a benzoquinone (single ring) or napthaquinone (double ring).

Without being tied to an exact mechanism, it is believed that the compounds referred to above function by initiating and propagating a free radical mechanism, thereby producing active chemical species that selectively attack abnormal cell structures. In Cancer Research 36 (1978), 1745 to 1750, Bachur et al described possible free radical mechanisms in connection with the known biological actions of quinone-containing anti-cancer drugs. It is postulated that these drugs may generate oxygen-dependent free radicals such as superoxide or hydroxyl radicals. In the present invention, it is possible that the above-mentioned compounds serve as catalysts for a redox-recycling mechanism which continuously generates free radicals such as superoxides. The free radicals, or their by-products, may selectively act in destroying cancer cells or the effect of viruses and the like. Alternatively, one or more of the other mechanisms mentioned above may be relevant or dominant.

Carminic acid has been used in the laboratory for staining nucleic acids and, interestingly, its effect on DNA is inhibited by, inter alia, free radical scavengers (Lown et al., Bioorganic Chem. 8 (1979), 17 to 24.

A typical unit dose of the composition of the invention in solution or suspension is preferably about 2 mls. However, there may be used a range of from about 2.0 to about 5.0mls which is particularly useful when the composition is administered by injection. On the other hand, when the composition is administered orally an orally-acceptable diluent such as water may be used for convenient to bring the dose up to a drinkable level of say about 100 mls.

These doses can apparently be substantially independent of the bodyweight of the host animal and typically, administration e.g. by injection is about 3 times per week which in the case of cancer is until the tumour disappears. There may then follow a maintenance dose taken orally up to say four times a day.

According to another feature of the invention, the efficacy of the postulated free radical chain reaction mechanism may be enhanced through administration of iron or any other transition metal, especially copper. Yet another useful catalyst for the free radical mechanism is a (low concentration) polyunsaturated fatty acid, which is a long chain free carboxylic acid typically found in a lipid.

Generally, carminic acid etc., in therapeutically-effective concentrations as described herein, appear to stimulate the immune system. Moreover, carminic acid and other quinones may usefully be employed at the said concentrations along with a compound as defined in International Patent Application No. PCT/GB91/00517, that is:

one or more compounds of the general formula:

X - P

wherein P is trinitrophenyl and X is selected from OH, $NH_2$, halogen, a sulfo group, a carboxyl group, $OCH_3$ or a substituted or unsubstituted hydrazyl group of the formula:

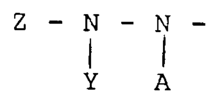

wherein A is hydrogen or an unpaired electron of the nitrogen atom, Y is hydrogen or an organic group and Z is an organic group, or Y and Z together with the adjacent nitrogen atom form a nitrogen-containing heterocycle; provided that when X is a substituted or unsubstituted hydrazyl group as aforesaid one of the $NO_2$ groups may be replaced by a sulfo group each at a concentration of about $10^{-3}$ moles/litre or below.

Compositions in accordance with the invention and their use will now be described by way of illustration only with reference to the following specific Examples.

Example 1

A composition for use in treating AIDS was prepared according to the following formulation:
Carminic acid
Double-distilled, de-ionized water,
balance to make a concentration of $10^{-6}$ moles/litre.

Example 2

In one pilot study, a thirty seven year old male was diagnosed as HIV positive by the standard ELISA test. When first examined in November, 1990, the patient had oral thrush, very severe herpes zooster of the left facial nerve with involvement of the left orbital region, hard bilateral cervical lymph nodes, and an enlarged liver and spleen. Thereafter, the patient was treated with carminic acid in a composition in accordance with Example 1 via subcutaneous injections. For five days, the patient received a single 1.0 ml injection per day. After six days, a similar five day course (one injection per day for five days) was repeated. After the fourth course (20 injections), the patient was in good general condition, was no longer anaemic, and the oral thrush, cervical lymph nodes and herpes zooster infection had cleared. The spleen and liver were normal and the patient has gained weight. Significantly, the patient's white blood count (WBC) had increased from 2,000 cells/$mm^{-3}$ to 12,400 cells/$mm^{-3}$, with a corresponding increase in haemoglobin (Hb) from 11.8 to 14.7 grams per decilitre.

Example 3

A composition for use in treating cancer was prepared according to the following formulation:
Carminic acid
Triple-distilled, de-ionized water,
balance to make a concentration of $10^{-15}$ moles/litre.

Example 4

P388 murine lymphonic cells were grown in a BDFI mouse. One injection of 5 ml of the formulation of Example 3 was administered subcutaneously. The mouse was still alive after a period of six months.

Example 5

AB, a 36 year old housewife was well until August, 1991, when she noticed a lymphnode enlargement in her neck associated with intraabdominal masses. Clinical assessment at that time confirmed generalised lymphnode enlargement and an enlarged spleen and liver. The spleen and the liver were 8 cm and 4 cm below costal margin respectively.

Histological and CT scan evaluations confirmed a diagnosis of low grade lymphoma involving lymphnodes, spleen, liver and bone marrow. She refused palliative chemotherapy. She was then put on a combination of carminic acid, quinhydrone, and tetrahydroxyquinone. In the formulation the three drugs were mixed in equal

proportions in sterile distilled deionised water at a concentration of $10^{-6}$ M. She received 2 mls of this formulation by subcutaneous injections using a clean sterile glass syringe on days 1, 3, 5 and 7. She was then put on a maintenance oral therapy of the same formulation for three months. In the oral therapy she took 100 mls of the above formulation four times a day on an empty stomach. She responded immediately and at the end of the third month of treatment she was in complete clinical remission. She was still in good health at the time this report was written (February, 1992).

Example 6

GJ, a 65 year old woman with progressive metastatic malignant melanoma involving right maxillary and ethmoid sinuses, and multiple skin legions was put on the protocol of Example 5 above on 22nd December, 1991, after failing to respond to both radiotherapy and chemotherapy. At the time of making this report was compiled, six weeks after the beginning of treatment, she is showing remarkable response to the treatment. All the peripheral multiple skin lesions have completely disappeared (the largest 4.5 x 4.5cm) and the sinus disease is undergoing neurosis and healing. The swelling and the redness have completely disappeared. She continues to make steady progress.

Example 7

RB, a 46 year old woman with metastatic cancer of the cervix stage 4 had extensive pelvic disease involving the left kidney and secondary deposits on the lumbar vertebrae. She was put on the same protocol as in Example 5 on 22nd December, 1991. When she was reevaluated six weeks after the initiation of the treatment she was found to be free from any pain and the tumour in the cervix was half the size. She continues to make an uneventful recovery.

Example 8

VW, a 27 year old woman was diagnosed in 1989 as having AIDS confirmed by the standard ELISA test. She presented on 10th October, 1991 with persistent fever, chronic diarrhoea, productive cough, severe oral thrush, and severe loss of weight. Clinical evaluation revealed a very sick patient who was severely anaemic and had generalised lymph node enlargement. She was wasted and had severe oral thrush associated with bleeding sores all over her month. A blood test at that time confirmed the anaemia, Hb 6.5g% (normal range 14.5 to 17.50g%), and low white blood cell count (WBC) of 2900 (normal range 4,500 to 5,800/$mm^3$). She was put on the same protocol as in Example 5 on 12th October, 1991, When she was reviewed eight weeks later all her symptoms had disappeared and she had put on weight. She was mildly anaemic, but had no lymphnode enlargement and no oral thrush. Her Hb was 13. 2g% and WBC count 4800/$mm^3$. She continues to make steady recovery.

While the invention has been described in part by reference to various preferred embodiments, those skilled in the art will appreciate that various modifications, substitutions, omissions and changes may be made without departing from the spirit and scope of the invention as defined by the claims which follow.

## Claims

1. A pharmaceutical composition, which composition comprises at least one quinone dissolved or dispersed in a liquid diluent or carrier at a concentration of about $10^3$ moles/litre or below.

2. A composition according to claim 1, wherein the or at least one quinone is:
   an anthraquinone glycoside;
   a compound of the formula:

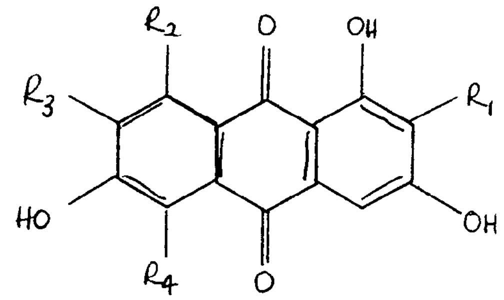

wherein $R_1$ is hydrogen, a sugar e.g. $-C_6H_{11}O_5$, hydroxy,

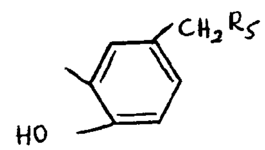

in which $R_5$ is $-CH_2NHAc$, $-CH_2OH$, $-CH(NH_2)CO_2H$ or $-CH_2NH_2$; $R_2$ is methyl or $-CO_2H$, $R_3$ is hydrogen, $-CO_2H$, $-NH_2$ or $-SO_3X$ in which X is H, Na or K; and
R4 is hydrogen or hydroxy.

3. A composition according to claim 2, wherein the or at least one quinone is:
   carminic acid;
   laccaic acid A, B, C, D or E, kermesic acid, ceroalbolinic acid, erythrolaccin or deoxyerythrolaccin;
   quinhydrone of the formula:

Or

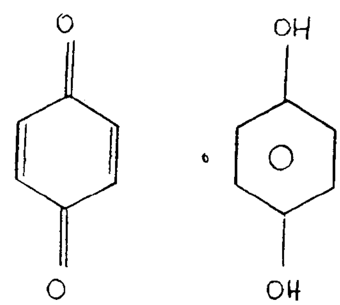

tetrahydroxyquinone of the formula:

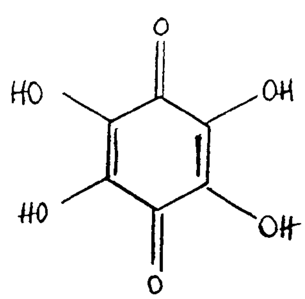

or a derivative thereof such as:

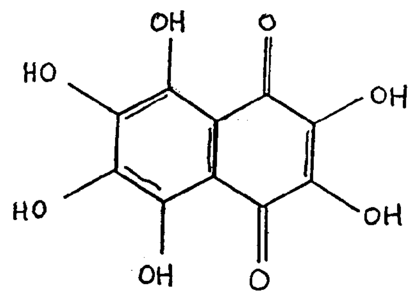

III(a)

2,3,5,6,7,8-hexahydroxy-naphthoquinone
or

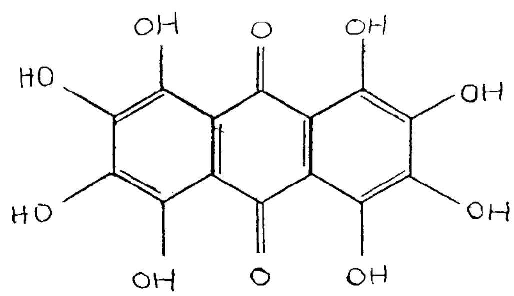

III(b)

1,2,3,4,5,6,7,8-octahydroxy-anthraquinone

4. A composition according to any one of the preceding claims, wherein there are at least two quinones.

5. A composition according to claim 4, wherein the quinones are present in at least approximately equal amounts.

6. A composition according to claim 4 or claim 5, comprising carminic acid, quinhydrone and/or tetrahydroxyquinone, preferably one comprising approximately equal amounts of carminic acid, quinhydrone and tetrahydroxyquinone.

7. A composition according to any one of the preceding claims, wherein the concentration of each quinone is from about $10^3$ to about $10^{-18}$ moles/litre, preferably from about $10^{-6}$ to about $10^{-15}$ moles/litre.

8. A composition according to any one of the preceding claims, wherein the diluent or carrier comprises water, polyethylene glycol, an oil such as arachis oil, or a liquid paraffin, preferably double- or triple-distilled deionized water.

9. A composition according to any one of the preceding claims, in unit dosage form and comprising from about 2 ml to about 5 ml, preferably about 2 ml, of composition.

10. A composition comprising at least one quinone dissolved or dispersed in a pharmaceutically-acceptable liquid medium at a concentration of from about $10^3$ to about $10^{-18}$ moles per litre for use in the therapy or prophylaxis of disease in human and non-human animals.

11. A composition according to any one of claims 1 to 9, for use in the therapy or prophylaxis of disease in human and non-human animals.

**12.** A composition according to claim 19 or claim 20 for use orally or parenterally.

**13.** A composition according to any one of the preceding claims for use in the treatment of cancer or in the treatment of virus infection, especially AIDS.

**14.** A composition according to any one of the preceding claims which includes:
one or more compounds of the general formula:

X - P

wherein P is trinitrophenyl and X is selected from OH, $NH_2$, halogen, a sulfo group, a carboxyl group, $OCH_3$ or a substituted or unsubstituted hydrazyl group of the formula:

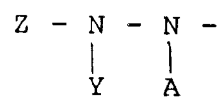

wherein A is hydrogen or an unpaired electron of the nitrogen atom, Y is hydrogen or an organic group and Z is an organic group, or Y and Z together with the adjacent nitrogen atom form a nitrogen-containing heterocycle; provided that when X is a substituted or unsubstituted hydrazyl group as aforesaid one of the $NO_2$ groups may be replaced by a sulfo group, each at a concentration of about $10^3$ moles/litre or below.

**15.** For use in the manufacture of a medicament a composition according to any one of the preceding claims.